Europäisches Patentamt

European Patent Office

Office européen des brevets

(11) Publication number: **0 214 443**
**A1**

## (12) EUROPEAN PATENT APPLICATION

(21) Application number: 86110512.0

(22) Date of filing: 30.07.86

(51) Int. Cl.⁴: **A61M 1/02** , F04B 43/08 , F04B 43/12

(30) Priority: 02.08.85 JP 169704/85

(43) Date of publication of application:
18.03.87 Bulletin 87/12

(84) Designated Contracting States:
CH DE FR GB IT LI SE

(71) Applicant: NIKKISO CO., LTD.
No. 43-2, Ebisu 3-chome
Shibuya-ku Tokyo(JP)

(72) Inventor: Oonishi, Yasutaka
c/o NIKKISO CO., LTD. No. 3-43-2, Ebisu
Shibuya-ku Tokyo(JP)
Inventor: Aizawa, Takeshi
c/o NIKKISO CO., LTD. No. 3-43-2, Ebisu
Shibuya-ku Tokyo(JP)

(74) Representative: Grättinger, Günter
Wittelsbacherstrasse 5 Postfach 16 49
D-8130 Starnberg(DE)

(54) A transfusion pump.

(57) An improved transfusion pump is disclosed in which a transfusion tube (50), a plurality of finger plates (52) each contacted with one side of the transfusion tube (50) for sequentially pressing and occluding the transfusion tube (50), and a reception plate (54) opposite to the finger plates (52) for holding the other side of said transfusion tube (50) are arranged, each of the finger plates (52) being contacted in a predetermined differential phase with eccentric cams (56) mounted to a rotary shaft (58) while on their either side being provided slidably with guide elements (66), and the rotary shaft (58) at its one end being connected coaxially to a driving motor (64).

FIG. 1

FIG.2

## A TRANSFUSION PUMP

This invention relates to a transfusion pump, more particularly to the improvement in a peristaltic driving mechanism of a peristaltic finger pump.

In general, transfusion pumps have been utilized in a medical field for maintaining health of patients having no or little ability of taking oral nutrition but need for transfusion or other nutrient management, for parenteral replenishment with nutrients or for parenteral administration of pharmaceutical fluids. Recently, increased cardio-surgery for aged persons and seriously diseased infants has required nutritious management through transfusion in an aspiratory and circulatory aspects during and/or after surgical operation, as well as utmost attention to a transfusion fluid especially for infants after the cardio-surgery. Thus, the importance for transfusion pumps of such type has increased. For these purposes, a compact transfusion pump of light weight is desired which has high precision of infusion and requires lower consumption of an electric power. Recently, there have been needed especially transfusion pumps of such a type that may be used for a long time even without available power supply. From this point of view, critical requirement for the transfusion pumps resides in that power saving and long operability may be achieved by means of an integrated battery.

As the transfusion pumps there have hitherto been known pumps of a roller type which utilizes an elastic transfusion tube made of vinylchloride or silicone to be squeezed successively by a rotating roller for ensuring a pumping action, and of a finger type which has a plurality of finger plates for squeezing the transfusion tube sequentially in a feeding direction of fluid thereby to achieve the pumping action. Among these pumps, the latter finger type is superior in the infusion accuracy and thus has been widely utilized.

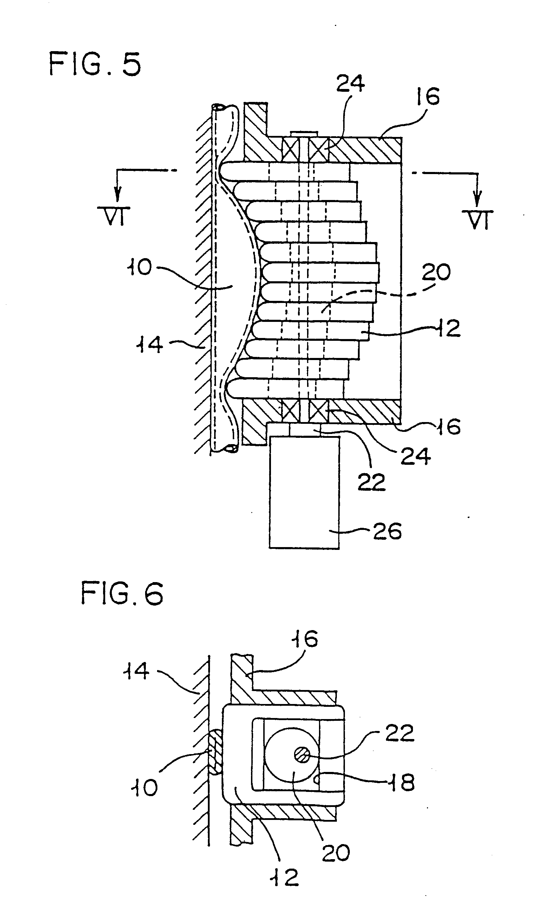

Figures 5 and 6 in the accompanying drawings illustrates a conventional pump of the finger type. In Figure 5, numerical reference 10 represents a transfusion tube which is provided at its one side with a plurality of peristaltic finger plates 12 and at its other side with a reception plate 14 opposite to the finger plates for sequentially occluding or opening the tube 10. The finger plates 12 are supported by a holder 16 and each provided with an elongated hole 18 in which an eccentric cam 20 with a rotary shaft 22 is arranged to generate a required peristaltic movement of the finger plates 12 (Fig. 6). In the illustrated embodiment, the eccentric cam 20 is formed of twelve pieces which are arranged adjacent to each other in a phase difference of 30° (= 360°/12 pieces). The rotary shaft 22 is supported to the holder 16 through bearings 24, 24 and at its one end is connected to a driving motor 26. Thus, the rotating movement of the eccentric cam 20 may be converted to a linear movement of the finger plates 12 for sequentially urging the transfusion tube 10 in a constant orientation, thereby to ensure the liquid-feeding within the tube 10. Thus, a series of the finger plates 12 may cause a pulsative movement represented by a formula $x = \ell(1 - \cos \omega t)$ wherein $\ell$ represents a degree of eccentricity and $\omega$ represents an angular velocity of the motor 26.

The transfusion pump thus constructed, however, is subject to a larger friction between the eccentric cam 20 and the finger plates 12 or between the finger plates and the holder 16 due to its construction. In order to overcome the friction, and additional torque is required in addition to a necessary torque of the motor 26 for urging the transfusion tube 10. Consequently, the conventional transfusion pump has required a higher electric power for driving the motor 26, resulting not only in difficulty of long operation of the pump with its integrated battery but also in a large size and a heavy weight of the pump with, for example, a battery of a higher capacity, as well as an increased manufacturing cost.

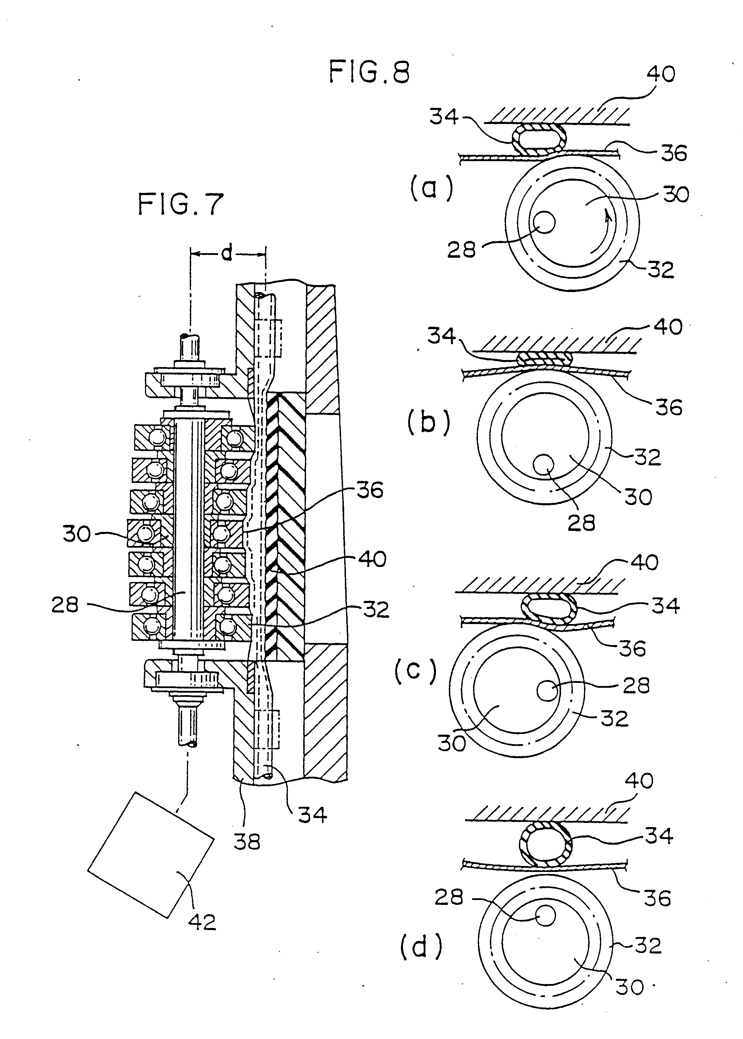

In view of a disadvantage of the increased torque of the driving motor in the finger type pump as described-above, there has also been proposed a transfusion pump, as shown in Fig. 7, in which a plurality of eccentric cams 30 are fixed onto a rotary shaft 28 so as to be deviated at a predetermined angle from each other while each of the eccentric cams 30 over its circumference is fitted with a bearing 32, between which bearing 32 and a transfusion tube 34 is arranged an elastic sheet element 36 for preventing the bearing 32 from squeezing the transfuison tube 34 directly. In Fig. 7, reference 38 represents a guide for guiding the transfusion tube 34 and securing the rotary shaft 28, reference 40 represents a reception plate for securing the transfusion tube 34 and reference 42 stands for a driving motor connected to one end of the rotary shaft 28.

In the rotary transfusion pump thus constructed, arrangement of the elastic sheet element between the bearing 32 and the transfusion tube 34 is essential. If the bearing 32 at its circumference should squeeze the transfusion tube directly, then the bearing 32 could be rotated together with the eccentric cam 30, so that the transfusion tube 34 may be pressed and moved longitudinally, thereby to lower its efficient squeezing action. Such type of the transfuion pump, in contrast to the finger type transfusion pump as illustrated in Fig. 5, has an advantage of fewer elements of generating friction, so that a lower driving torque is necessitated for the driving motor 42. In this type of rotary transfusion pump, however, the elastic sheet element 36 is essential, which cannot completely eliminate the longitudinal movement of the transfusion tube 34 due to co-rotation of the bearing 32 with the eccentric cam 30 and hence the longitudinal pressing action on the transfusion tube 34 via the elastic sheet element 36, as shown in Figs. 8(a) -(d). Further, in contrast to the finger type transfusion pump of pressing the tube planarly, the rotary type must squeeze the tube arcuately and thus an outer diameter of the transfusion tube for the efficient squeezing action should be limited to sufficiently less than that of the bearing. Still further, the rotary transfusion pump has a less distance between an end face of the guide 38 abutting the transfusion tube 34 and an axis of the rotary shaft 28, so that the driving motor 42 is difficult to be arranged on the axis of the rotary shaft 28. For this reason, the design of reducing the torque does not permit the driving torque of the motor 42 to be transmited onto the rotary shaft 28 due to their arrangement. If an outer diameter of the bearing 32 is made larger in order to facilitate the arrangement of the driving motor 42 on the axis of the rotary shaft 28, for example, a total size of the pump itself should be large and also a moment required for squeezing the transfusion tube 34 should be higher with need for a higher torque of the driving motor 42.

Accordingly, an object of the invention is to provide a compact transfusion pump of a light weight having a high precision of infusion and consuming a lower electric power. For this purpose, a further object of the invention is to utilize the advantageous mechanism of the finger type pump of pressing the transfusion tube planarly and to avoid the disadvantageous friction between the elements by separating the finger plates from the eccentric cams, thereby to reduce a torque loss due to the friction between the finger plates and the eccentric cams for achieving the compactness, the light weight and the power saving of the transfusion pump.

In order to achieve the above objects, the invention provides a transfusion pump comprising a transfusion tube, a plurality of finger plates each contacted with one side of said transfusion tube for sequentially pressing and occluding said transfusion tube, and a reception plate opposite to said finger plates for holding the other side of said transfusion tube, each of said finger plates being contacted in a predetermined differential phase with eccentric cams mounted to a rotary shaft while on their either side being provided slidably with guide elements, and said rotary shaft at its one end being connected coaxially to a driving motor.

Thus, in accordance with the invention, the plurality of the eccentric cams are rotated on the rotary shaft at an equal eccentricity and in an equal angular phase difference and provided in contact thereto with retreatable finger plates which are restricted for their moving direction by the guide elements to prevent swinging movement of the finger plates, thereby to utilize the advantage of the conventional transfusion pump of a finger type efficiently and to eliminate its disadvantage for providing a considerably improved efficient transfusion pump.

In the transfusion pump according to the invention, each eccentric cam over its circumference may be fitted coaxially with a bearing through which said eccentric cam is contacted with the finger plate in order to more efficiently reduce the torque loss due to the friction between the components.

As described herein-above, in accordance with the invention, the plurality of finger plates for pressing and occluding the transfusion tube may be contacted with the latter only for the pressing action by the design of the eccentric cams, thereby to solve the problems of the conventional finger type pumps, to significantly reduce the torque loss due to the friction between the components, to ensure the proper arrangement of the driving motor for rotating the eccentric cams, to enable the stable and reliable continuous operation with the integrated battery, and thus to improve the performance of the transfusion pump considerably.

The invention will now be described for its preferred embodiments with reference to the accompanying drawings.

Figure 1 is a sectional side view in part of one embodiment of the transfusion pump according to the invention;

Figure 2 is a sectional view taken along the line II-II in Fig. 1;

Figure 3 is a sectional view in part of another embodiment of the transfusion pump according to the invention;

Figure 4 is a sectional view taken along the line IV-IV in Fig. 3;

Figure 5 is a sectional view in part of one example of the conventional transfusion pumps;

Figure 6 is a sectional view taken along the line VI-VI in Fig. 5;

Figure 7 is a sectional view of another example of the conventional transfusion pumps; and

Figures 8 (a) -(d) are schematic views of illustrating operation of the eccentric cams acting on the transfusion tube of the transfusion pump, as shown in Fig. 7.

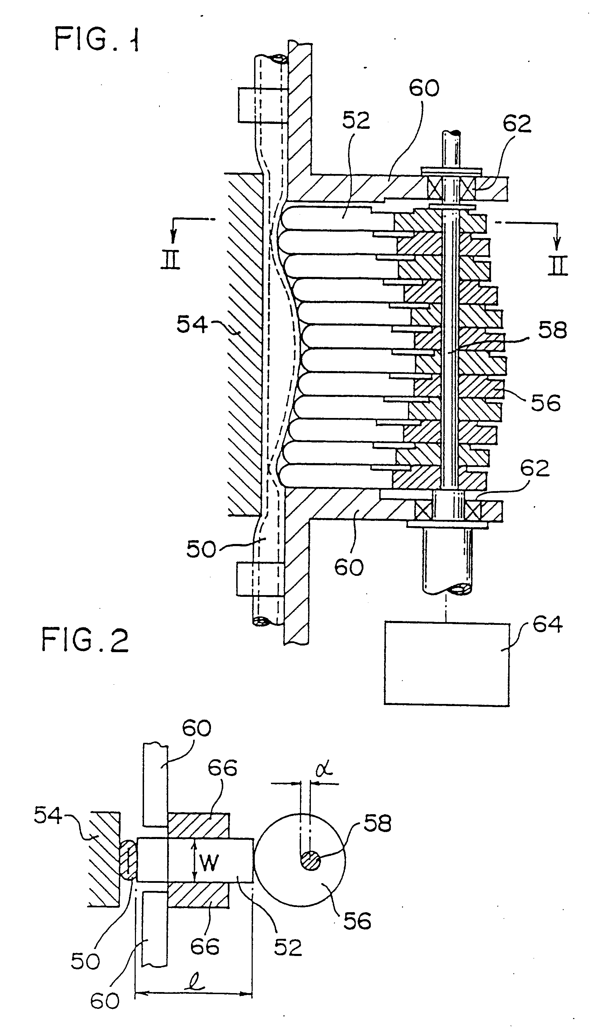

Figures 1 and 2 illustrate one embodiment of the transfusion pump according to the invention, wherein reference 50 represents a transfusion tube, on one side of which are arranged a plurality of finger plates 52 for sequentially pressing and occluding the tube 50 while on the other side of the tube 50 and opposite to the finger plates 52 is arranged a reception plate 54 for supporting the tube 50. This construction is basically identical to that of the conventional finger type pump. In the pump of this embodiment, however, twelve finger plates 52 of each predetermined length $l$ are arranged in parallel, each of which at its one end is contacted with an eccentric cam 56 having a predetermined eccentricity $\alpha$. These eccentric cams 56 are secured to a rotary shaft 58 at a phase difference of 30 ° (= 360 ° /12 pieces). The rotary shaft 58 at its either end is supported rotatably through bearings 62, 62 to a holder 60 for holding the finger plates 52 and at its one end is connected to a driving motor 64. The plurality of finger plates 52 at their both ends are supported slidably by guide elements 66, 66 for ensuring proper straight movement without swinging, as shown in Fig. 2.

The operation of the transfusion pump of this embodiment thus constructed will be described herein-after. If the driving motor 64 rotates at a given angular velocity $\omega$ , then the rotary shaft 58 and the eccentric cams 56 fixed thereto also rotate at the same angular velocity. With rotation of the eccentric cams 56 the finger plates 52 contacted therewith at their front ends are moved at the phase difference of 30 ° in the form of a sine wave represented by a formula $\alpha$ ( 1 -cos$\omega$t ) for pressing the transfusion tube 50. As a result, the transfusion tube 50 is pressed and occluded sequentially from an upstream side to a downstream side for allowing a transfusion fluid contained therein to be fed in a constant direciton. In this case, each eccentric cam 56 urges the respective finger plate 52 only when the latter moves in the direction of pressing the transfusion tube 50, while the finger plate 52 is pushed back toward the rotary shaft 58 when the tube 50 is restored by the fluid filled therein. Thus, in this embodiment the torque loss on the driving motor 64 due to the friction between the components may be considerably reduced in comparison with the conventional finger type pump. Further, a width W of the finger plate 52 may be set to, for example, a tube-distortion width of sufficiently pressing the transfusion tube 50. In this regard, the width W may be reduced, leading to the significant reduction of the torque loss compared with that of the conventional finger pump.

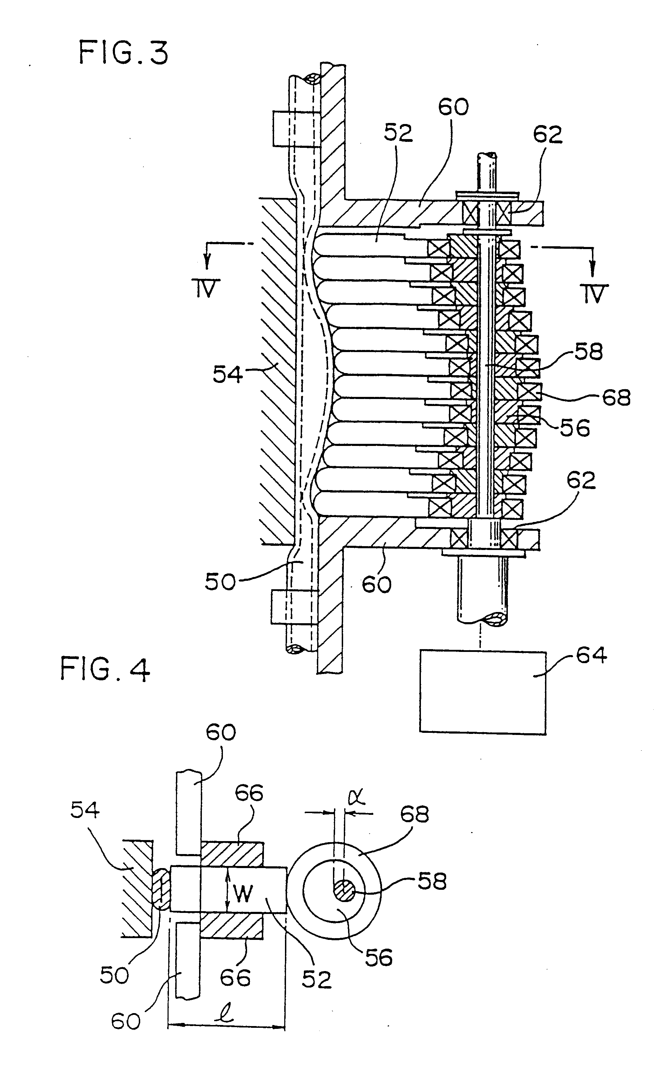

Figures 3 and 4 illustrate another embodiment of the transfusion pump according to the invention, wherein the eccentric cam 56 is modified differently from that in the previous embodiment of Figs.

1 and 2. Namely, in this embodiment the eccentric cam 56 has a reduced outer diameter and over its circumference is provided coaxially with a bearing 68. Otherwise the construction is identical to that of the previous embodiment and the same reference are used for representing the same components. Accordingly, the detailed description of the same components is omitted for simplicity.

According to this embodiment, the eccentric cams 56 are contacted through the bearing 68 to the finger plates 52, so that the torque loss due to the friction between the components may be much more reduced, resulting in the effective power saving of the driving motor 64.

As described herein-above, in the transfusion pump according to the invention starting from the defective construction of the finger type pump, the eccentric cams are separated from the finger plates but contacted with the latter only when pressing the transfusion tube, while each finger plate has an enough width W for pressing the tube and a suitable length $l$ and is moved straight properly by the guide elements, so that the torque loss due to the friction between the components may be considerably reduced and that the coaxial connection of the driving motor with the rotary shaft for rotating the eccentric cams may be possible, resulting in the compactness, the light weight and the power saving of the transfusion pump having the high precision.

Although the invention has been described herein-above with its preferred embodiments, it will be appreciated that many variations and modifications may be made without departing from the spirit and the scope of the invention.

## Claims

1. A transfusion pump comprising a transfusion tube, a plurality of finger plates each contacted with one side of said transfusion tube for sequentially pressing and occluding said transfusion tube, and a reception plate opposite to said finger plates for holding the other side transfusion tube, each of said finger plates being contacted in a predetermined differential phase with eccentric cams mounted to a rotary shaft while on their either side being provided slidably with guide elements, and said rotary shaft at its one end being connected coaxially to a driving motor.

2. A transfusion pump according to claim 1, wherein each eccentric cam over its circumference is fitted coaxially with a bearing through which said eccentric cam is contacted with the finger plate.

FIG. 1

FIG. 2

FIG.3

FIG.4

FIG. 5

FIG. 6

FIG.8

FIG.7

## DOCUMENTS CONSIDERED TO BE RELEVANT

EP 86110512.0

| Category | Citation of document with indication, where appropriate, of relevant passages | Relevant to claim | CLASSIFICATION OF THE APPLICATION (Int. Cl.4) |
|---|---|---|---|
| Y | JP - A2 - 58-165 868 (YOKOGAWA DENKI)  <br> * Fig. 1,2,5-7 * | 1,2 | A 61 M  1/02 <br> F 04 B 43/08 <br> F 04 B 43/12 |
| Y | GB - A - 1 182 908 (POLYMETRON AG)  <br> * Totality, especially page 2, lines 31-33, 50-55 * | 1,2 | |
| Y | US - A - 4 479 797 (SUSUMU KOBAYASHI et al.)  <br> * Totality, especially fig. 1; column 1, lines 55-63 * | 1 | |
| A | DE - A1 - 3 202 251 (MGVG MED. GER.)  <br> * Totality * | 1 | |
| A | FR - A - 2 492 902 (SECAN)  <br> * Totality * | 1 | |
| A | GB - A - 1 081 818 (DISTILLERS COMP.)  <br> * Totality, especially page 2, lines 98-104 * | 1 | |
| A | DE - A1 - 3 104 873 (TERUMO CORP.)  <br> * Totality * | 1 | |
| A | US - A - 4 482 347 (A. BORSANYI)  <br> * Totality * | 1 | |

**TECHNICAL FIELDS SEARCHED (Int. Cl.4)**

A 61 M  1/00

A 61 M  5/00

F 04 B 43/00

The present search report has been drawn up for all claims

| Place of search | Date of completion of the search | Examiner |
|---|---|---|
| VIENNA | 18-12-1986 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82